# EUROPEAN PATENT SPECIFICATION

(11) **EP 4 091 505 B1**
(45) Date of publication and mention of the grant of the patent: **31.07.2024**
(21) Application number: 21740894.7
(22) Date of filing: 13.01.2021
(51) Int. Cl.: A47C 27/15, A61N 1/14

(54) **RESTING ELEMENT WITH CONNECTION TO THE EARTH CONNECTION OF THE DWELLING**
RUHEELEMENT MIT ANSCHLUSS AN DEN ERDUNGSANSCHLUSS DER WOHNUNG
ÉLÉMENT DE REPOS AVEC UNE CONNEXION À LA PRISE DE TERRE DE L'HABITATION

(30) Priority: 16.01.2020 ES 202030057 U
(43) Date of publication of application: 23.11.2022
(73) Proprietor: Jiménez Castillo, Maria Rosario, 18101 Belicena (ES)
(72) Inventor: Jiménez Castillo, Maria Rosario, 18101 Belicena (ES)
(74) Representative: Sahuquillo Huerta, Jesús
(86) International application number: PCT/ES2021/070011
(87) International publication number: WO 2021/144489

(56) References cited:
- EP-A1- 2 103 233
- EP-A1- 3 417 744
- WO-A1-2017/140931
- CN-A- 108 099 292
- CN-A- 108 784 121
- KR-A- 20180 071 901
- US-A1- 2015 265 063

## Description

### Object of the invention

The object of the present report is an element of rest with connection to the earth connection of the house, consisting essentially of a bed base of laminated vaporised beech wood, with 100% natural water-based varnish, and mattress with latex core, made of emulsion of the resin of the *Heveabrasilensis* tree, 100% natural, beaten and baked in oven at 120 degrees, without chemical additives, which incorporates an anti-humidity ionising sheet of vaporised and pressed coconut 100% natural, with a central layer of pink salt rocks and white quartz, an antibacterial mesh of bamboo thread and a cover made of 70% cashmere and 30% organic cotton fabric, with a 4 cm filling in at least the upper part of the mattress, made of 100% natural wool and organic graphite and silver fabric and an aluminium plate associated with an earthing device; and where, in turn, the present invention incorporates an addition of graphene at the edge of the bed base and at the bottom of the mattress, together with three platinum antennas as ionisers, and finally, a new mesh fabric of gold, silver and/or graphite filament, which increase its conductive capacity.

### Background to the invention

Nowadays, one of the fields that is becoming more and more important in the domestic sphere is the fact of how to achieve a better physical and/or mental state through a correct rest.

The applicant is in turn the holder of the invention protected by Spanish patent ES 2 574 355, which describes a rest set for the recovery of the geoelectric field, consisting of a bed base made of laminated vaporised beech wood, with 100% natural water-based varnish, and a mattress with a latex core, made of emulsion from the resin of the *Hevea Brasilensis* tree, 100% natural, beaten and baked in an oven at 120 degrees, without chemical additives, incorporating an anti-humidity ionising sheet of 100% natural steamed and pressed coconut, with a central layer of pink salt rocks and white quartz, an anti-bacterial mesh of bamboo thread and a cover of 70% cashmere and 30% organic cotton fabric, with a 4 cm filling in at least the upper part of the mattress, of 100% natural wool and organic graphite and silver fabric and an aluminium plate associated with an earthing device.

The applicant is also aware of international application PCT/ES2007/000013 which describes a therapeutic rest bed, comprising a bed base with beechwood slats attached to the frame in mobile groups of two or three slats, depending on the areas of the body to which they correspond, by means of pivoting parts, shock-absorbing supports, height-adjustable supports and clamps, a 100% natural latex mattress with holes and a coconut fibre sheet, a mattress cover with organic carbon mesh connected to an earthing conductor, a mattress cover with another organic carbon mesh, a blanket with a merino wool cover with a thermo-regulating effect and a pillow, also made of 100% natural latex.

Likewise, he is aware of the existence of utility model ES 1 058 937 which describes a bed with slatted bed base and mattress; of a kind comprising a bed base provided with transverse slats for the support of the mattress and a mattress intended to rest on those slats to form a sleeping surface, wherein:
- the bed base comprises: an outer frame, an inner frame, a group of slats positioned in correspondence with the head support zone and mounted on the inner frame by means of elastic pieces of natural rubber; a group of slats positioned in correspondence with the shoulder support zone and mounted on the inner frame by means of elastic pieces of natural rubber attached to shock-absorbing supports; a group of double slats, with adjustable flexion, located in the back support area and mounted on the inner frame by means of elastic pieces of natural rubber; a group of slats located in the lumbar support area and mounted on the inner frame by means of elastic pieces of natural rubber attached to height-adjustable supports; and, a group of slats located in the leg support area and supported on the inner frame by means of elastic pieces of natural rubber;
- the mattress comprises: an inner core composed of a prismatic block of 100% natural latex with a plurality of holes of different diameters that define zones of different resistance and a lower layer of coconut fibre; an inner cover of cotton fabric; and an outer cover with an inner padding of merino wool and an organic carbon fabric to which an outer earthing conductor is connected, the free end of which ends in a piece of conductive material, such as copper or similar, for earthing.

With the aim of continuously improving the solutions to this problem, the applicant has made a series of improvements to the invention protected by Spanish patent ES 2 574 355, namely the presence of a new system or means of energy discharge through the connection between the elements that make up the rest element with connection to the earth of the dwelling, through an improved universal connection system adapted for discharge, which will allow this energy to be safely discharged to the earth, avoiding overloads in the system.

### Description of the invention

The technical problem solved by the present invention is to obtain a means of rest (a bed or similar) that includes means for increasing the electrical conductivity of a resting element. For this purpose, the rest element with connection to the earth connection of the dwelling, which is the subject of the present report, comprising a toper or mattress topper, a bed base and a latex mattress; said bed base being made of laminated vaporised beech wood, varnished with 100% natural water-based varnish, and a mattress made of a latex core, composed of an emulsion of the resin of the *Hevea brasilensis* tree, 100% natural, beaten and baked in an oven at 120 degrees Celsius, without any chemical additives, which, without any chemical additives, is made of latex, without any chemical additives, which, in addition, incorporates an ionising and anti-humidity layer of steamed and pressed 100% natural coconut, with a central layer of pink salt rocks and white quartz rocks, as well as an antibacterial mesh of bamboo thread; and because the said mattress also has a cover made of a fabric made of 70% cashmere and 30% organic cotton, with a filling that covers at least the upper part of the mattress with a thickness of about four centimetres, made of 100% natural wool. The toper has a means of connection to the mattress and this, in turn, to the bed base, which is finally connected to the earth connection of the home through a universal connection system that incorporates a safety system to avoid overloading the line; and because the cover incorporates a mesh of gold, silver and organic graphite and silver filament which, together with the connection with the rest of the elements, improves its residual discharge and insulating capacity.

Thanks to its design, the rest element recommended here uses materials that serve to alleviate the technical problem posed, and where, one of the aspects of vital importance is the presence of a body or graphene element on the edge of the bed base or bottom of the mattress, which will be connected to the toper or mattress topper; and in this way they will be connected in turn with the bed base that includes the earth connection connected to the earth connection of the house.

The sheath will have a mesh of gold, silver, and graphite filament to increase its conductive capacity, which, together with the connection to the rest of the elements, improves its residual discharge and insulating capacity.

The connection to the earth connection of the dwelling will preferably be made via a universal connection system (improved and adapted for discharge), incorporating two plastic connections and with only the earth connected, with a safety system for overloading the line.

The internal connections allow all the elements (topper, mattress, and bed base) to be continuously connected to each other, and their "joint" is connected to the earth of the house, therefore, with only one earth connection, the discharge is extended.

### Brief description of the figures

The following is a very brief description of a series of drawings which help to better understand the invention, and which relate expressly to an embodiment of the invention which is presented as a non-limiting example of the invention.

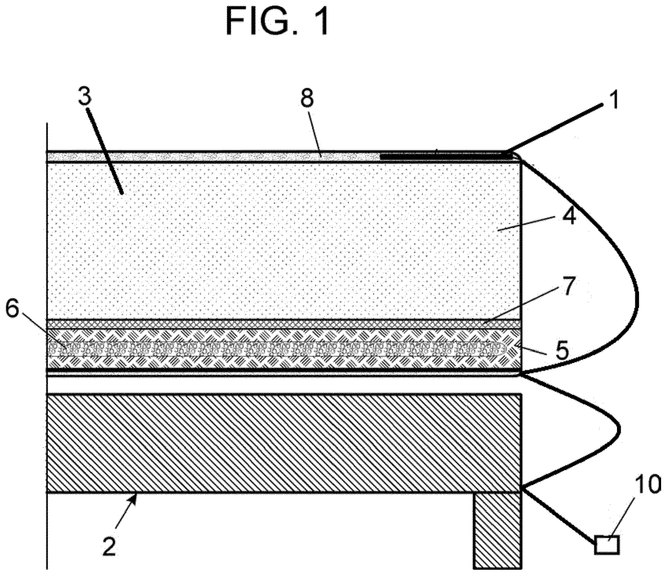

FIG 1. Shows a schematic view of the earthed sleeping element of the dwelling.

### Detailed description of an embodiment of the invention

The attached figures show a preferred embodiment of the invention. More specifically, the rest element with earth connection of the dwelling, which is the subject of the present report, essentially comprises a toper or mattress topper (1), a bed base (2) and a latex mattress (3); said bed base (2) being made of laminated vaporised beech wood, varnished with 100% natural water-based varnish.

The mattress (3) is made of a core (4) of latex, composed of an emulsion of the resin of the *Hevea brasilensis* tree, 100% natural, beaten and baked in an oven at 120 degrees, without any chemical additives, which also incorporates an ionising and anti-humidity layer (5) of steamed and pressed 100% natural coconut, incorporates an ionising and anti-humidity film (5) of 100% natural steamed and pressed coconut, and inside it has a central layer (6) of pink salt rocks and white quartz rocks, as well as an antibacterial mesh (7) of bamboo thread.

Preferably, the ionising and moisture-proof coconut sheet (5) is about 3 centimetres thick and is located at the bottom of the mattress, while the antibacterial mesh (7) made of bamboo thread, also preferably, is incorporated into the mattress (3) between the latex core (4) and the ionising and moisture-proof coconut sheet (5).

In any case, the mattress (3) also has a cover (8) made of a fabric made of 70% cashmere and 30% organic cotton, with a filling that covers at least the upper part of the mattress with a thickness of about 4 centimetres, made of 100% natural wool.

In addition, the sheath (8) incorporates a mesh of gold, silver and organic graphite and silver filament, which, together with the connection with the rest of the elements, improves its residual discharge and insulating capacity.

In a preferred embodiment, the toper (1) will have a means of connection to the mattress (3) and this, in turn, to the bed base (2), which will finally be connected to the earth connection (10) of the dwelling through a universal connection system that incorporates a safety system to avoid overloading the line.

## Claims

1. Resting element with connection to the earth connection of the dwelling, of the type comprising a toper or mattress topper (1), a bed base (2) and a latex mattress (3); the said bed base (2) being made of laminated steamed beech wood, varnished with 100% natural water-based varnish, and a mattress (3) made of a latex core (4), composed of an emulsion of the resin of the *Hevea brasilensis* tree, 100% natural, beaten and baked in an oven at 120 degrees Celsius, without any chemical additive, which, without any chemical additive, is made of latex, without any chemical additives, which, in addition, incorporates an ionising and anti-humidity layer (5) of 100% natural steamed and pressed coconut, with a central layer (6) of pink salt rocks and white quartz rocks, as well as an antibacterial mesh (7) of bamboo thread; and because said mattress (3), in addition, has a cover (8) made of a fabric made of 70% cashmere and 30% organic cotton, with a filling that, at least, covers the upper part of the mattress with a thickness of about 4 centimetres, made of 100% natural wool and wherein the toper (1) has a means of connection to the mattress (3) and this, in turn, to the bed base (2), which is finally connected to the earth connection (10) of the home through a universal connection system that incorporates a safety system to avoid overloading the line; and **characterized in that** the sheath (8) incorporates a mesh of gold, silver and organic graphite and silver filament which, together with the connection with the rest of the elements, improves its residual discharge and insulating capacity.

2. Resting element with connection to the earth connection of the house according to claim 1, wherein the ionising and anti-humidity sheet (5) of coconut is about 3 centimetres thick and is placed in the lower part of the mattress, while the antibacterial mesh (7) of bamboo thread, also preferred, is incorporated in the mattress (3) between the core (4) of latex and the said ionising and anti-humidity sheet (5) of coconut.

## Patentansprüche

1. Liegeelement mit Anschluss an den Erdanschluss der Wohnung, bestehend aus einem Topper oder einer Matratzenauflage (1), einem Lattenrost (2) und einer Latexmatratze (3); wobei der Lattenrost (2) aus gedämpftem Buchenschichtholz besteht, das mit 100% natürlichem Lack auf Wasserbasis lackiert ist, und eine Matratze (3), die aus einem Latexkern (4) besteht, der aus einer Emulsion des Harzes des *Hevea* brasilensis-Baumes besteht, die 100% natürlich ist, geschlagen und in einem Ofen bei 120 Grad Celsius gebacken wird, ohne jeglichen chemischen Zusatzstoff, die ohne chemische Zusätze aus Latex besteht und außerdem eine ionisierende und feuchtigkeitshemmende Schicht (5) aus 100% natürlicher, gedämpfter und gepresster Kokosnuss mit einer zentralen Schicht (6) aus rosa Salzsteinen und weißen Quarzsteinen sowie ein antibakterielles Netz (7) aus Bambusfäden enthält; und weil die Matratze (3) außerdem einen Bezug (8) aus einem Gewebe aus 70 % Kaschmir und 30 % Bio-Baumwolle hat, mit einer Füllung, die zumindest den oberen Teil der Matratze mit einer Dicke von etwa 4 cm bedeckt und aus 100 % natürlicher Wolle besteht, und wobei der Topper (1) ein Mittel zur Verbindung mit der Matratze (3) und diese wiederum mit dem Bettkasten (2) hat, der schließlich mit dem Erdanschluss (10) des Hauses durch ein universelles Verbindungssystem verbunden ist, das ein Sicherheitssystem enthält, um eine Überlastung der Leitung zu vermeiden; und **dadurch gekennzeichnet, dass** die Ummantelung (8) ein Netz aus Gold, Silber und organischem Graphit und Silberdraht enthält, das zusammen mit der Verbindung mit den übrigen Elementen seine Restentladung und Isolierfähigkeit verbessert.

2. Liegeelement mit Anschluss an den Erdanschluss des Hauses nach Anspruch 1, **dadurch gekennzeichnet, dass** die ionisierende und feuchtigkeitshemmende Kokosnussfolie (5) etwa 3 cm dick ist und im unteren Teil der Matratze angeordnet ist, während das ebenfalls bevorzugte antibakterielle Netz (7) aus Bambusfaden in die Matratze (3) zwischen dem Latexkern (4) und der ionisierenden und feuchtigkeitshemmenden Kokosnussfolie (5) eingearbeitet ist.

## Revendications

1. Elément de repos relié à la prise de terre de l'habitation, du type comprenant un toper ou surmatelas (1), un sommier (2) et un matelas en latex (3) ; ledit sommier (2) étant réalisé en bois de hêtre stratifié étuvé, verni avec un vernis à l'eau 100% naturel, et un matelas (3) constitué d'une âme en latex (4), composée d'une émulsion de la résine de l'arbre *Hevea brasilensis,* 100% naturelle, battue et cuite au four à 120 degrés Celsius, sans aucun additif chimique, qui, sans aucun additif chimique, est en latex, sans aucun additif chimique, qui, en outre, incorpore une couche ionisante et anti-humidité (5) de noix de coco 100 % naturelle, étuvée et pressée, avec une couche centrale (6) de roches salines roses et de roches de quartz blanc, ainsi qu'une maille antibactérienne (7) en fil de bambou ; et parce que ledit matelas (3) possède en outre une housse (8) constituée d'un tissu composé de 70% de cachemire et de 30% de coton biologique, avec un rembourrage qui couvre au moins la partie supérieure du matelas avec une épaisseur d'environ 4 centimètres, en laine 100% naturelle et dans lequel le surmatelas (1) possède un moyen de connexion au matelas (3) et celui-ci, à son tour, au sommier (2), qui est finalement connecté à la prise de terre (10) de l'habitation à travers un système de connexion universel qui incorpore un système de sécurité afin d'éviter la surcharge de la ligne ; et **caractérisé par le fait que** la gaine (8) incorpore un maillage de filaments d'or, d'argent et de graphite organique et d'argent qui, avec la connexion avec le reste des éléments, améliore sa capacité de décharge résiduelle et d'isolation.

2. Élément de repos relié à la prise de terre de la maison selon la revendication 1, dans lequel la feuille ionisante et anti-humidité (5) de noix de coco a une épaisseur d'environ 3 centimètres et est placée dans la partie inférieure du matelas, tandis que la maille antibactérienne (7) en fil de bambou, également préférée, est incorporée dans le matelas (3) entre le noyau (4) de latex et ladite feuille ionisante et anti-humidité (5) de noix de coco.
